# FASCICULE DE BREVET EUROPEEN

(11) **EP 2 643 671 B1**
(45) Date de publication et mention de la délivrance du brevet: **02.03.2016**
(21) Numéro de dépôt: 11790759.2
(22) Date de dépôt: 24.11.2011
(51) Int. Cl.: A61B 5/0215, G01L 9/12, A61B 5/00

(54) **CAPTEUR DE PRESSION MINIATURISE**
MINIATURISIERTER DRUCKSENSOR
MINIATURIZED PRESSURE SENSOR

(30) Priorité: 24.11.2010 FR 1059678
(43) Date de publication de la demande: 02.10.2013
(73) Titulaire: Nanomade Concept, 31100 Toulouse (FR)
(72) Inventeur: MOUCHEL LA FOSSE, Eric, Singapore 550544 (SG); SONGEON, Lionel, F-31170 Tournefeuille (FR)
(74) Mandataire: Ipside
(86) Numéro de dépôt international: PCT/EP2011/070973
(87) Numéro de publication internationale: WO 2012/069604

(56) Documents cités:
- EP-A2- 1 635 158
- WO-A1-03/018307
- DE-A1-102005 035 022
- US-A1- 2007 074 577
- KLARA E. MUEGGENBURG ET AL: "Elastic membranes of close-packed nanoparticle arrays", NATURE MATERIALS, vol. 6, no. 9, 1 septembre 2007 (2007-09-01), pages 656-660, XP055002270, ISSN: 1476-1122, DOI: 10.1038/nmat1965
- HERRMANN J ET AL: "Nanoparticle films as sensitive strain gauges", APPLIED PHYSICS LETTERS, AMERICAN INSTITUTE OF PHYSICS, US, vol. 91, no. 18, 30 October 2007 (2007-10-30), pages 183105-183105, XP012099977, ISSN: 0003-6951, DOI: 10.1063/1.2805026

## Description

L'invention concerne un capteur de pression miniaturisé. Elle est particulièrement utile pour mesurer la pression d'un fluide véhiculé dans un conduit et plus particulièrement pour mesurer une distribution spatiale, axiale ou circonférentielle de cette pression dans ce conduit.

La mesure de la pression, relative ou absolue et plus particulièrement de la distribution spatiale ou temporelle de la pression, d'un fluide véhiculé dans un conduit, permet de déterminer de nombreux paramètres relatifs aux conditions d'écoulement dudit fluide et de déterminer des propriétés intrinsèques du fluide véhiculé dans le conduit, propriétés telles que la viscosité dudit fluide ou son débit instantané.

La connaissance de ces paramètres est notamment utile lorsque le fluide véhiculé est un fluide corporel, humain ou animal, tel que le sang ou l'urine. Par exemple, la mesure de la pression d'un fluide ou de la distribution spatiale de cette pression lors de la circulation extracorporelle, en pontage, de ce fluide dans un conduit instrumenté, permet de recueillir des informations sur les propriétés de ce fluide et ses conditions de circulation sans effectuer de prélèvement de celui-ci. Selon un autre exemple d'application, particulièrement avantageux, la mesure de la pression, de sa distribution spatiale ou temporelle peut être réalisée sur un conduit intracorporel tel qu'un vaisseau sanguin.

Les dispositifs de mesure de la pression, non intrusifs, connus de l'art antérieur, consistent en un corps d'épreuve dont la déformation est mesurée à l'aide d'une jauge, dite de contrainte, ledit corps d'épreuve devant être couplé mécaniquement avec le conduit de sorte à ce que la déformation du conduit, sous l'effet de la pression du fluide qu'il véhicule, soit transmise audit corps d'épreuve. La demande de brevet internationale WO2006122750 décrit un tel capteur de pression apte à être implanté sur un conduit intracorporel tel qu'un vaisseau sanguin.

Les documents WO03/018307, "Nanoparticle films as sensitive strain gauges" Herrmann J et al et "Elastic membranes of close-packed nanoparticle arrays" Klara E. Mueggenburg et al décrivent l'utilisation d'une assemblée de nanoparticules comme détecteur de contraintes (déformations). La nécessité d'obtenir un couplage mécanique entre le conduit et le corps d'épreuve du capteur modifie localement la réponse mécanique du conduit, voire, modifie la forme de ce conduit et peut avoir des conséquences sur les conditions d'écoulement du fluide. Ainsi, de tels capteurs ne permettent pas de mesurer une distribution circonférentielle ou axiale de la pression sur de petites distances.

Pour résoudre les insuffisances de l'art antérieur, l'invention propose un dispositif pour mesurer la pression d'un fluide véhiculé dans un conduit, caractérisé en ce qu'il comprend :
a. une première électrode ;
b. une seconde électrode ;
c. une assemblée de nanoparticules conductrices ou semi-conductrices en contact avec les deux électrodes ;
d. un dispositif de mesure délivrant une information proportionnelle à une propriété électrique de l'assemblée de nanoparticules, laquelle propriété est mesurée entre la première et la seconde électrode, ladite propriété électrique étant sensible à la distance entre les nanoparticules de l'assemblée ;
e. l'assemblée de nanoparticules, liée mécaniquement à un substrat souple, étant en liaison mécanique avec le fluide véhiculé, par le conduit de telle sorte que les distances entre les nanoparticules de ladite assemblée soient modifiées par une variation de pression dans ledit fluide; le substrat souple est apte à être lié au conduit de telle sorte que l'assemblée de nanoparticules soit à l'interface entre ledit substrat souple et une paroi du conduit.
Ainsi, l'utilisation de nanoparticules permet de réaliser un capteur à la fois très sensible et de petite dimension, adaptable à de nombreuses situations de mesure. En effet, ainsi placée sur un substrat souple, ladite assemblée de nanoparticules est apte à être utilisée comme corps d'épreuve et à mesurer directement un effort qui lui est appliqué, indépendamment de la rigidité du support sur lequel le dispositif est placé, ou, elle peut être rapportée sur un corps d'épreuve dont elle peut mesurer les déformations, ces deux modes d'utilisation pouvant être combinés.

Cette configuration permet notamment de conserver l'assemblée de nanoparticules comme corps d'épreuve du capteur de pression, le substrat n'ayant alors qu'une influence très réduite sur les conditions d'écoulement du fluide.

D'autres avantages de l'utilisation des nanoparticules ressortiront des modes de réalisation avantageux, décrits ci-après, lesquels peuvent être considérés individuellement ou selon toute combinaison techniquement opérante.

Dans tout le texte, le verbe « lier » et le terme « liaison » expriment une relation fonctionnelle entre deux éléments, plus particulièrement les termes « liaison mécanique » expriment une transmission d'effort entre les éléments considérés que cette transmission soit directe ou que le flux d'effort traverse également d'autres éléments.

Avantageusement, le dispositif objet de l'invention comprend un des moyens aptes à lier mécaniquement le substrat souple sur lequel est placée l'assemblée de nanoparticules à la paroi d'un conduit véhiculant un fluide. Ce mode de réalisation est particulièrement avantageux pour pourvoir rapporter de manière provisoire le dispositif objet de l'invention sur un conduit, par exemple sur un conduit intracorporel.

Selon un mode de réalisation du dispositif objet de l'invention, les moyens de liaison mécanique entre le substrat souple et le conduit comprennent une bague apte à s'ouvrir par une charnière pour y insérer le conduit, le substrat souple formant la charnière de cette bague. Ce mode de réalisation permet de proposer un dispositif de mesure sous la forme d'un clip dont les branches sont de constitution rigide, faciles à manipuler, sans que la rigidité des branches n'influe sur la réponse du conduit, du fait de la souplesse de la charnière, tout en permettant une mesure fine des variations, notamment temporelles, de la pression du fluide véhiculé dans ledit conduit.

Avantageusement, les nanoparticules sont constituées d'or et le substrat souple est constitué d'un matériau bio-résorbable. Ainsi, le dispositif objet de l'invention peut être placé sur un conduit intracorporel et s'éliminer naturellement.

Selon un mode de réalisation particulier du dispositif objet de l'invention, la propriété électrique mesurée de l'assemblée de nanoparticules est la capacité électrique de cette assemblée. Ainsi la mesure de la variation de cette propriété électrique, et par suite de la pression du fluide véhiculé par le conduit, peut être réalisée sans contact et à distance, notamment par l'intermédiaire d'un circuit résonant, et sans que le dispositif ne soit connecté à un circuit par une connexion filaire. Ce mode de réalisation est particulièrement avantageux lorsque le dispositif objet de l'invention est utilisé pour la mesure de la pression d'un fluide véhiculé dans un conduit intracorporel.

L'invention concerne également, un conduit instrumenté apte à véhiculer un fluide et comprenant un dispositif selon l'un quelconque des modes de réalisation décrits ci-avant.

Selon un mode de réalisation de ce conduit objet de l'invention, le dispositif de mesure est lié à la paroi du conduit, ledit conduit formant le corps d'épreuve. Ce mode de réalisation est particulièrement adapté pour mesurer de la pression d'un fluide lors de sa circulation dans un circuit extracorporel. Le conduit instrumenté est alors simplement placé dans ce circuit.

Selon un autre mode de réalisation, compatible avec le précédent, le dispositif de mesure de la pression est lié à la paroi interne du conduit en contact avec le fluide véhiculé. Ce mode de réalisation est plus particulièrement avantageux lorsque l'assemblée de nanoparticules du dispositif de mesure de la pression, est utilisée directement comme corps d'épreuve. Dans ce cas le conduit instrumenté permet de délivrer une information très précise sur la distribution de pression dans l'écoulement et d'en déduire des informations tant sur les propriétés du fluide que sur ses conditions d'écoulement.

L'invention sera maintenant plus précisément décrite dans le cadre de ses modes de réalisation préférés, nullement limitatifs, et des figures 1 à 6, dans lesquelles :
- la figure 1 représente en perspective et en vue de dessus un capteur élémentaire faisant partie du dispositif objet de l'invention selon l'un de ses modes de réalisation ;
- la figure 2, illustre en coupe le principe de fonctionnement d'un capteur élémentaire selon un exemple de réalisation du dispositif objet de l'invention, où le capteur élémentaire est sensible à une sollicitation sensiblement parallèle à la direction d'empilement des couches de nanoparticules, figure 2A sans sollicitation du capteur élémentaire, figure 2B lorsque le capteur élémentaire est sollicité mécaniquement ;

- la figure 3 montre en coupe et en bout, deux variantes de mise en oeuvre d'un dispositif objet de l'invention sur un conduit, figure 3A sur la paroi interne du conduit, figure 3B sur la paroi externe dudit conduit ;
- la figure 4 illustre le fonctionnement d'un capteur élémentaire, dit continu, en perspective et vue de dessus ;
- la figure 5 est un exemple vu en bout et en coupe de l'utilisation d'un capteur élémentaire continu tel que représenté figure 4, pour la réalisation d'un dispositif selon un mode de réalisation de l'invention, ledit capteur élémentaire continu étant installé à l'intérieur d'un conduit, figure 5A, ou à l'extérieur du conduit, figure 5B ;
- la figure 6 représente selon une vue en bout et en coupe, un exemple de réalisation du dispositif objet de l'invention mettant en oeuvre une bague munie d'une zone charnière ;
- et la figure 7 est un exemple de réalisation vue en perspective et en bout d'un conduit instrumenté selon un mode de réalisation de l'invention.

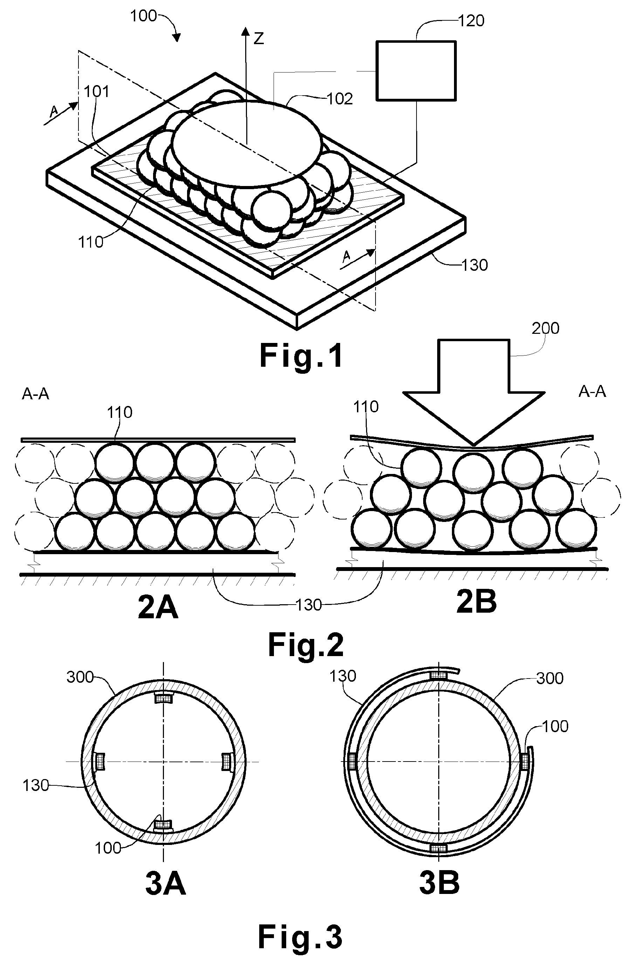

Figure 1, selon un exemple de réalisation, le dispositif de mesure objet de l'invention comprend un capteur élémentaire (100) comportant une première électrode (101) et une seconde électrode (102), une assemblée de nanoparticules (110) électriquement conductrices ou semi-conductrices liées par un ligand électriquement isolant (non représenté). Selon cet exemple de réalisation, ladite assemblée comprend au moins deux couches de nanoparticules (110) empilées selon une direction d'empilement (Z). Les deux électrodes (101, 102) sont en contact électrique avec l'assemblée de nanoparticules. Des moyens de mesure (120) permettent de mesurer une propriété électrique de l'assemblée de nanoparticules, par exemple sa résistance électrique. L'ensemble constitué des deux électrodes et de l'assemblée de nanoparticules est placé sur un substrat (130), et est avantageusement recouvert d'un film isolant (non représenté). À déformation équivalente, un tel capteur élémentaire est au moins 100 fois plus sensible qu'une jauge d'extensométrie piézorésistive classique montée en pont dit de wheastone. La dimension des nanoparticules (110) est comprise entre 2.10⁻⁹ mètres, ou nanomètres, et peut atteindre 50 µm de sorte que l'épaisseur du capteur élémentaire est très faible, au plus de l'ordre de 0,2 mm mais peut être réduite à 0,02 µm (10⁻⁶ mètres) selon la dimension des nanoparticules et le nombre de couches empilées.

Figure 2A, vue en coupe et sans sollicitation, les nanoparticules de l'assemblée du capteur élémentaire sont ordonnancées selon un empilement sensiblement compact. Figure 2B, lorsqu'une sollicitation mécanique (200) est appliquée à l'assemblée de nanoparticules, la distance entre toutes ou partie des nanoparticules (110) de l'assemblée est modifiée, ce qui modifie les propriétés électriques de ladite assemblée. La mesure d'une propriété électrique sensible à cette distance permet ainsi de déterminer l'intensité de cette sollicitation mécanique. La sollicitation mécanique peut être une force, une pression ou une déformation imposée au dispositif objet de l'invention, soit directement à celle-ci, soit par l'intermédiaire d'un corps d'épreuve. Le substrat (130) est dit souple car il ne s'oppose pas à la modification de la distance entre les nanoparticules (110) lorsque l'assemblée est sollicitée directement.

Figure 3, selon un exemple de réalisation du dispositif objet de l'invention, celui-ci comprend un ou plusieurs capteurs élémentaires (100) comprenant chacun une assemblée de nanoparticules, répartis sur la circonférence d'un conduit (300), à l'intérieur duquel est véhiculé un fluide. Chacun de ces capteurs élémentaires fonctionne selon le principe illustré figure 2, c'est-à-dire qu'il est sensible à une sollicitation mécanique appliquée sensiblement parallèlement à la direction d'empilement des nanoparticules de l'assemblée.

Figure 3A, selon un exemple de réalisation, les capteurs élémentaires (100) sont placés à l'intérieur du conduit (300) en contact avec le fluide véhiculé. Ainsi, chacun de ces capteurs mesure directement la pression appliquée, là où il se trouve. Les capteurs élémentaires (100) étant de très faible épaisseur, leur présence ne perturbe pas l'écoulement dans le fluide

Figure 3B, selon un autre exemple de réalisation, les capteurs élémentaires (100) sont placés à l'extérieur du conduit (300), entre la paroi dudit conduit et un substrat (130) de forme sensiblement homothétique à la forme du conduit. La forte sensibilité des capteurs élémentaires permet d'utiliser un substrat (130) dont la rigidité est sensiblement équivalente à celle de la paroi du conduit (300) et sa présence ne modifie pas sensiblement les conditions d'écoulement du fluide dans ledit conduit.

Ce substrat souple (130) permet également de connecter les capteurs élémentaires à la paroi du conduit (300), que ceux-ci soient placés à l'intérieur ou à l'extérieur dudit conduit. Selon un premier exemple de réalisation, le substrat souple est essentiellement élastique et la connexion avec le conduit (300) est réalisée à la manière d'un clip, par une expansion de celui-ci pour le connecter à l'extérieur du conduit ou en le rétreignant pour le connecter à l'intérieur du conduit (300). Alternativement, le substrat souple présente une rigidité suffisamment faible pour être enroulé autour du conduit. Dans ce dernier cas un collage peut parfaire cette connexion.

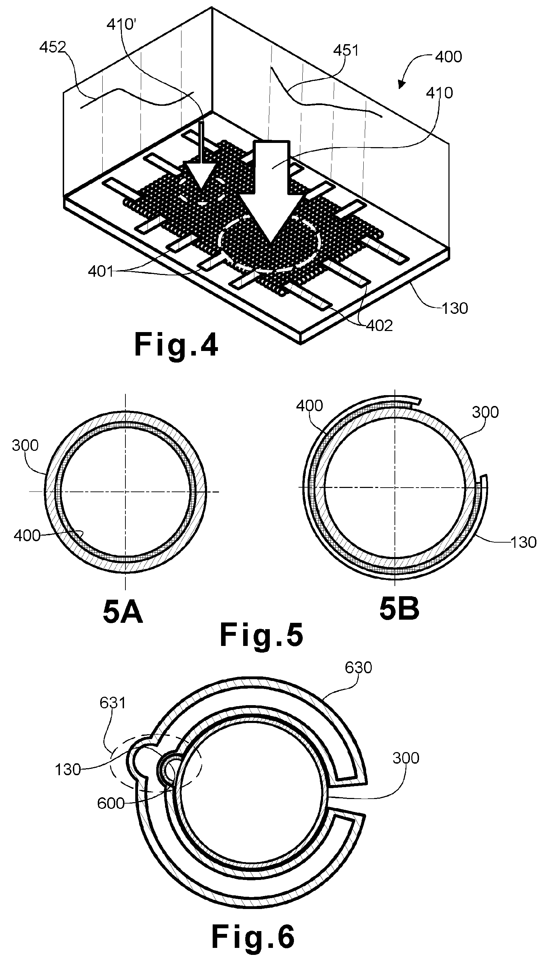

Figure 4, selon un autre exemple de réalisation d'un capteur élémentaire, dit capteur élémentaire continu (400) celui-ci comprend une pluralité de premières électrodes (401) et une pluralité de secondes électrodes (402) en contact avec une assemblée de nanoparticules, déposées sur un substrat (130) Ainsi, en mesurant les variations d'une propriété électrique de l'assemblée de nanoparticules lorsque celle-ci est sollicitée par un système de sollicitations (410, 410'), et en procédant par une série de mesures associant deux à deux, selon les diverses combinaisons possibles d'électrodes (401, 402) entre ces deux pluralités, il est possible d'établir un profil de variation (451, 452) de ladite propriété électrique sur toute la surface de l'assemblée et, par suite, d'en déduire des informations sur le système de sollicitations (410, 410') à l'origine de cette variation de la propriété électrique.

Figure 5, ce capteur élémentaire continu (400) peut-être mis en oeuvre, à l'intérieur d'un conduit (300), figure 5A, ou à l'extérieur de celui-ci, figure 5B, pour obtenir une cartographie de la pression du fluide véhiculé. Figure 5A, selon un exemple de réalisation, le capteur élémentaire continu (400) est déposé directement sur la paroi interne du conduit ledit conduit étant constitué d'un matériau souple, jouant le rôle de substrat. Le dépôt peut être réalisé par des techniques de dépôt capillaire convectif, ou en encore des techniques de lithographie douce par tamponnage. Selon un exemple de réalisation le dépôt est réalisé à plat, sur le flan apte à constituer le conduit, ledit flan étant ensuite roulé et soudé afin de réaliser ledit conduit. Alternativement, le capteur peut être déposé par lithographie douce ou par dépôt capillaire convectif, sur la paroi du conduit déjà roulé, celui-ci étant retroussé de sorte à exposer sa paroi interne à l'extérieur.

Figure 5B selon un autre mode de réalisation le capteur élémentaire continu (400) est déposé sur un substrat (130) de forme homothétique au conduit puis placé en contact avec la paroi externe dudit conduit (300). Ce mode de réalisation est plus particulièrement utile pour placer ledit capteur sur un conduit qui ne peut être ouvert, par exemple sur un conduit intracorporel tel qu'une veine. Un tel capteur peut ainsi être placé sur ce conduit intracorporel à la manière d'un clip, sans traumatisme pour ledit conduit. La sensibilité élevée du capteur élémentaire continu (400) permet d'utiliser un substrat (130) souple et mince qui ne perturbe pas l'écoulement et peut être laissé à l'intérieur du corps sans occasionner de gêne.

Pour de telles applications intracorporelles, le substrat peut être avantageusement constitué d'un matériau bio-résorbable, tel qu'un polymère d'acide lactique ou d'autres polymères bio-résorbables. Les nanoparticules peuvent avantageusement être constituées d'or et déposées sous une forme colloïdale facilement éliminée par l'organisme. Avec ce type de nanoparticules, combinées à des ligands non toxiques et un substrat bio-résorbable, l'ensemble du capteur (100, 400) est facilement éliminé par l'organisme au bout d'un temps défini. Un tel capteur peut ainsi être placé sur un conduit intracorporel de sorte à réaliser des mesures pendant un temps déterminé sans occasionner de gêne pour le patient, et s'éliminer naturellement sans nécessiter d'intervention chirurgicale pour son retrait.

Figure 6, selon une variante de réalisation, le dispositif objet de l'invention comprend un corps d'épreuve (630) apte à être placé sur un conduit (300) à la manière d'un clip. Ce corps d'épreuve (630) comprend une zone charnière (631) déformable qui est utilisée comme substrat pour un capteur élémentaire (600) comprenant une assemblée de nanoparticules. Le corps d'épreuve est en pratique la charnière, zone très souple, dont le capteur élémentaire (600) mesure la déformation. Pour ce type d'application, une seule couche de nanoparticules est nécessaire, le capteur (600) peut donc être extrêmement fin. La rigidité du capteur en dehors de la zone charnière (631) permet de manipuler facilement celui-ci, par exemple pour effectuer successivement des mesures en plusieurs points d'un conduit, notamment intracorporel. Le capteur élémentaire, placé sur la charnière (631) est protégé de toute dégradation au cours de cette manipulation.

Les figures 2 à 6, par commodité de représentation, ne représentent pas les connexions filaires du capteur élémentaire vers les moyens de mesure (120). Ces connexions peuvent être, au moins partiellement, réalisées sur le substrat (130) en les déposant sur celui-ci par des techniques de lithographie ou par des techniques de micro ou nano-impression notamment mettant en oeuvre des encres électriquement conductrices.

Selon un mode de réalisation particulier, la propriété électrique mesurée de l'assemblée de nanoparticules est la capacité électrique de cette assemblée. Chaque paire de nanoparticules électriquement conductrices, séparées par un ligand électriquement isolant constitue un nano-condensateur, dont la capacité est fonction, notamment, de la distance entre lesdites nanoparticules. La variation de capacité entre les électrodes (101, 102) est définie par la mise en série/parallèle de toutes les capacités entre les nanoparticules de l'assemblée. Cette configuration offre la possibilité de pouvoir lire la mesure à distance au moyen de protocoles du domaine des radiofréquences bien connus de l'art antérieur. Ainsi, aucune connexion filaire avec le capteur n'est nécessaire ce qui est particulièrement avantageux dans le cas d'applications intracorporelles dudit capteur, où cette caractéristique, combinée à la capacité de bio-résorption du dispositif, permet d'implanter un ou plusieurs dispositifs conformes à ce mode de réalisation de l'invention sur un conduit intracorporel afin de réaliser des mesures et un suivi de ces mesures sur une période donnée, sans occasionner de gêne pour le patient.

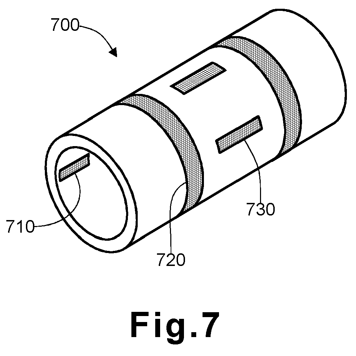

Figure 7, l'invention concerne également un conduit instrumenté (700) sur lequel sont installés plusieurs capteurs élémentaires. Ceux-ci peuvent être installés sur la paroi interne du conduit (710) ou sur la paroi externe dudit conduit. Les capteurs élémentaires (710, 720, 730) peuvent correspondre à l'un quelconque des modes de réalisation du dispositif de mesure objet de l'invention et exposé ci-avant. À titre d'exemple, non limitatif, ledit conduit instrumenté (700) peut comporter, sur sa face interne, un ou plusieurs capteurs élémentaires (710) directement sensibles à la pression. II peut comporter en paroi externe un ou plusieurs capteurs élémentaires (730) sensibles à la déformation longitudinale du conduit et un ou plusieurs capteurs élémentaires sensibles à la déformation radiale ou circonférentielle du conduit instrumenté (700). Selon ce mode de réalisation les capteurs élémentaires (710, 720, 730) sont adaptés aux caractéristiques du conduit et combinés de sorte à délivrer une information précise et sélectionnée soit sur les conditions d'écoulement du fluide soit sur les propriétés du fluide véhiculé. Ainsi, le conduit instrumenté peut être inséré dans un circuit afin de délivrer ces informations. Le conduit instrumenté (700) peut avantageusement être constitué d'un matériau bio-résorbable, qui combiné avec des capteurs élémentaires (710, 720, 730) comportant des nanoparticules et des ligands adaptés, permet d'insérer ledit conduit instrumenté dans un circuit de circulation intracorporelle, celui-ci étant éliminé naturellement.

La description ci-avant illustre clairement que par ses différentes caractéristiques et leurs avantages, la présente invention atteint les objectifs qu'elle visait. En particulier, elle permet de mesurer une distribution spatiale et temporelle de pression sur un conduit, notamment un conduit de circulation intracorporelle d'un fluide. Le capteur objet de l'invention ne nécessite pas de percer ledit conduit pour en mesurer la pression interne, évitant ainsi tout risque d'épanchement du fluide véhiculé consécutif à la mesure de pression.

## Revendications

1. Dispositif (100, 600, 710, 720, 730) pour mesurer la pression d'un fluide véhiculé dans un conduit (300, 700), comprenant :
a. une première électrode (101);
b. une seconde électrode (102) ;
c. une assemblée de nanoparticules conductrices (110) ou semi-conductrices en contact avec les deux électrodes ;
d. un dispositif de mesure (120) délivrant une information proportionnelle à une propriété électrique de l'assemblée de nanoparticules, laquelle propriété est mesurée entre la première (101) et la seconde électrode (102), ladite propriété électrique étant sensible à la distance entre les nanoparticules (110) de l'assemblée ;
e. **caractérisé en ce que** l'assemblée de nanoparticules (110), liée mécaniquement à un substrat souple (130), est en liaison mécanique avec le fluide véhiculé dans le conduit, de telle sorte que les distances entre les nanoparticules de ladite assemblée soient modifiées par une variation de pression dans ledit fluide ; et **en ce que** le substrat souple (130) est apte à être lié au conduit (300) de telle sorte que l'assemblée de nanoparticules soit à l'interface entre ledit substrat souple (130) et une paroi du conduit (300).

2. Dispositif selon la revendication 1, **caractérisé en ce qu'**il comprend un des moyens (630) aptes à lier mécaniquement le substrat souple (130) sur lequel est placée l'assemblée de nanoparticules à la paroi d'un conduit (300) véhiculant un fluide.

3. Dispositif selon la revendication 2, **caractérisé en ce que** les moyens de liaison mécanique entre le substrat souple et le conduit comprennent une bague (630) apte à s'ouvrir par une charnière (631) pour y insérer le conduit (300), le substrat souple (130) formant partie de la charnière de cette bague.

4. Dispositif selon la revendication 2, **caractérisé en ce que** les nanoparticules (110) sont constituées d'or et que le substrat souple (130) est constitué d'un matériau bio-résorbable.

5. Dispositif selon la revendication 1, **caractérisé en ce que** la propriété électrique est la capacité électrique de l'assemblée de nanoparticules.

6. Conduit instrumenté (700) apte à véhiculer un fluide **caractérisé en ce qu'**il comprend un dispositif de mesure selon la revendication 1.

7. Conduit selon la revendication 6, **caractérisé en ce que** le dispositif de mesure (710, 720, 730) est lié à la paroi du conduit, ledit conduit formant corps d'épreuve.

8. Conduit selon la revendication 6, **caractérisé en ce que** le dispositif (710) est lié à la paroi interne du conduit en contact avec le fluide véhiculé.

## Patentansprüche

1. Vorrichtung (100, 600, 710, 720, 730) zur Druckmessung eines in einer Leitung (300, 700) transportierten Fluids, welche umfasst:
a. eine erste Elektrode (101);
b. eine zweite Elektrode (102);
c. eine Gruppe von leitenden bzw. halbleitenden Nanopartikeln (110), die mit den beiden Elektroden in Kontakt sind;
d. eine Messvorrichtung (120), die eine zu einer elektrischen Eigenschaft der Gruppe von Nanopartikeln proportionale Information abgibt, wobei diese Eigenschaft zwischen der ersten (101) und der zweiten Elektrode (102) gemessen wird und in Hinblick auf die Distanz zwischen den Nanopartikeln (110) der Gruppe empfindlich ist;
e. **dadurch gekennzeichnet, dass** die Gruppe von Nanopartikeln (110), die mechanisch mit einem weichen Substrat (130) verbunden ist, mit dem in der Leitung transportierten Fluid derart mechanisch verbunden ist, dass sich die Abstände zwischen den Nanopartikeln dieser Gruppe durch eine Druckvariation in diesem Fluid ändern und dadurch, dass sich das weiche Substrat (130) an die Leitung (300) binden kann, sodass sich die Gruppe von Nanopartikeln an der Trennfläche zwischen dem weichen Substrat (130) und einer Leitungswand (300) befindet.

2. Vorrichtung nach Anspruch 1, **dadurch gekennzeichnet, dass** sie eins der Mittel (630) umfasst, die das weiche Substrat (130) mechanisch binden können, auf dem die Gruppe von Nanopartikeln an der Wand einer ein Fluid transportierenden Leitung (300) angebracht ist.

3. Vorrichtung nach Anspruch 2, **dadurch gekennzeichnet, dass** die mechanischen Verbindungsmittel zwischen dem weichen Substrat und der Leitung einen Ring (630) umfassen, der sich durch ein Scharnier (631) öffnen kann, um die Leitung (300) aufzunehmen, wobei das weiche Substrat (130) einen Teil dieses Ringscharniers bildet.

4. Vorrichtung nach Anspruch 2, **dadurch gekennzeichnet, dass** die Nanopartikel (110) aus Gold bestehen und das weiche Substrat (130) aus einem bioresorbierbaren Material ist.

5. Vorrichtung nach Anspruch 1, **dadurch gekennzeichnet, dass** es sich bei der elektrischen Eigenschaft um die elektrische Kapazität der Gruppe von Nanopartikeln handelt.

6. Instrumentierte Leitung (700), die ein Fluid transportieren kann, **dadurch gekennzeichnet, dass** sie eine Messvorrichtung nach Anspruch 1 umfasst.

7. Leitung nach Anspruch 6, **dadurch gekennzeichnet, dass** die Messvorrichtung (710, 720, 730) an die Leitungswand gebunden ist, wobei die Leitung einen Druckprobenkörper bildet.

8. Leitung nach Anspruch 6, **dadurch gekennzeichnet, dass** die Vorrichtung (710) an die Innenwand der Leitung in Kontakt mit dem transportierten Fluid gebunden ist.

## Claims

1. A device (100, 600, 710, 720, 730) to measure the pressure of a fluid carried in a conduit (300, 700), comprising:
a. a first electrode (101);
b. a second electrode (102);
c. an assembly of conducting nanoparticles (110) or semiconducting nanoparticles in contact with the two electrodes;
d. a measuring device (120) that provides information proportional with an electrical property of the assembly of nanoparticles, which property is measured between the first (101) and second electrode (102), wherein said electrical property is sensitive to the distance between the nanoparticles (110) of the assembly;
e. **characterized in that** the assembly of nanoparticles (110), joined mechanically to a flexible substrate (130), is mechanically joined with the fluid carried in the conduit, so that the distances between the nanoparticles of said assembly are modified by a variation of pressure in said fluid; and **in that** the flexible substrate (130) is suitable for being joined to the conduit (300) so that the assembly of nanoparticles is at the interface between said flexible substrate (130) and a wall of the conduit (300).

2. A device according to claim 1, **characterized in that** it comprises one of the means (630) suitable for mechanically joining the flexible substrate (130) on which is placed the assembly of nanoparticles to the wall of a conduit (300) carrying a fluid.

3. A device according to claim 2, **characterized in that** the mechanical joining means between the flexible substrate and the conduit comprise a ring (630) suitable for opening by a hinge (631) that allows the insertion of the conduit (300), wherein the flexible substrate (130) forms part of the hinge of said ring.

4. A device according to claim 2, **characterized in that** the nanoparticles (110) are made of gold and that the flexible substrate (130) is made of bio-resorptive material.

5. A device according to claim 1, **characterized in that** the electrical property is the electrical capacity of the assembly of nanoparticles.

6. An instrumented conduit (700) suitable for carrying a fluid **characterized in that** it comprises a measuring device according to claim 1.

7. A conduit according to claim 6, **characterized in that** the measuring device (710, 720, 730) is joined to the wall of the conduit, which conduit forms a test specimen.

8. A conduit according to claim 6, **characterized in that** the device (710) is joined to the internal wall of the conduit in contact with the carried fluid.
